# EUROPEAN PATENT SPECIFICATION

(11) **EP 2 938 656 B1**
(45) Date of publication and mention of the grant of the patent: **24.08.2016**
(21) Application number: 13821474.7
(22) Date of filing: 20.12.2013
(51) Int. Cl.: C08G 77/26, A61Q 5/00, A61K 8/898, C08G 77/32

(54) **MOLECULARLY IMPRINTED POLYMERS OF SOL-GEL TYPE AND THEIR USE AS ANTIDANDRUFF AGENT**
MOLEKULAR GEPRÄGTE POLYMERE VOM SOL-GEL-TYP UND IHRE VERWENDUNG ALS ANTISCHUPPENMITTEL
POLYMÈRES À EMPREINTES MOLÉCULAIRES DE TYPE SOL-GEL ET LEUR UTILISATION COMME AGENT ANTIPELLICULAIRE

(30) Priority: 26.12.2012 FR 1262784; 06.03.2013 US 201361773185 P
(43) Date of publication of application: 04.11.2015
(73) Proprietor: L'OREAL, 75008 Paris (FR)
(72) Inventor: GREAVES, Andrew, F-77700 Magny-le-Hongre (FR); RIBAUD, Christèle, F-94130 Nogent S/Marne (FR); MANFRE, Franco, F-94170 Le Perreux Sur Marne (FR); HAUPT, Karsten, F-60205 Compiègne (FR); TSE SUM BUI, Jeanne Bernadette, F-60205 Compiègne (FR)
(74) Representative: Rivière, François Armand
(86) International application number: PCT/EP2013/077790
(87) International publication number: WO 2014/102209

(56) References cited:
- US-A1- 2010 048 737
- ZHEN-JUAN DUAN ET AL: "Novel surface molecularly imprinted solâ gel polymer applied to the online solid phase extraction of methyl-3-quinoxaline-2-carboxylic acid and quinoxaline-2-carboxylic acid from pork muscle", ANALYTICAL AND BIOANALYTICAL CHEMISTRY, SPRINGER, BERLIN, DE, vol. 401, no. 7, 21 August 2011 (2011-08-21), pages 2291-2299, XP019953572, ISSN: 1618-2650, DOI: 10.1007/S00216-011-5329-0
- M. E. Díaz-García ET AL: "MOLECULARLY IMPRINTED POLYMERS" In: "Encyclopedia of Analytical Science (Second Edition)", 25 May 2005 (2005-05-25), Elsevier, XP055000717, ISBN: 978-0-12-369397-6 pages 172-182, DOI: 10.1016/B0-12-369397-7/00700-7, page 179, "Sol-Gel Molecular Imprinting" page 180, "Applications of Molecularly Imprinted Materials"
- DATABASE BIOSIS [Online] BIOSCIENCES INFORMATION SERVICE, PHILADELPHIA, PA, US; December 2005 (2005-12), DEANGELIS YVONNE M ET AL: "Three etiologic facets of dandruff and seborrheic dermatitis: Malassezia fungi, sebaceous lipids, and individual sensitivity", XP002715332, Database accession no. PREV200600189798 & JOURNAL OF INVESTIGATIVE DERMATOLOGY SYMPOSIUM PROCEEDINGS, vol. 10, no. 3, December 2005 (2005-12), pages 295-297, ISSN: 1087-0024

## Description

A subject matter of the invention is specific molecularly imprinted polymers of sol-gel type and also a cosmetic composition comprising them, and their use for eliminating or reducing dandruff of the scalp.

The appearance of dandruff, corresponding to a desquamative disorder of the scalp, is disagreeable both aesthetically and because of the annoyance which it causes (itching, redness, and the like), so that many people confronted with this problem to variable degrees wish to be rid of it efficiently and permanently.
Dandruff corresponds to an excessive and visible desquamation of the scalp resulting from excessively rapid multiplication of the epidermal cells. This phenomenon can be caused in particular by microtraumas of physical or chemical nature, such as excessively aggressive hair treatments, extreme climatic conditions, nervousness, diet, fatigue or pollution, but it has been demonstrated that dandruff conditions usually result from a disorder of the microflora of the scalp and more particularly from the excessive colonization of a yeast which belongs to the family of yeasts of the Malassezia genus (previously known as Pityrosporum ovale) and which is naturally present on the scalp.

The use is known, in order to combat dandruff, of topically applied antifungal agents. These agents are intended, by their antifungal power, to eliminate or control the multiplication of a resident yeast of the scalp belonging to the *Malassezia* genus and its variants (*M. ovalis, M. orbiculare, M. furfur, M. globosa,* etc.). Mention may be made, as antidandruff agents used for their antifungal action, of zinc pyrithione, piroctone olamine or selenium disulfide. These active agents can have an unfavourable impact on the overall quality of the scalp, including the dryness of the scalp, the color of the hair and the environment (The Antiseptic, 2004, 201(1), 5-8).

In the paper "Three Etiologic Facets of Dandruff and Seborrheic Dermatitis: Malassezia Fungi, Sebaceous Lipids, and Individual Sensitivity", Y.M. DeAngelis et al., J. Investig. Dermatol. Symp. Proc., 10, 295-297, 2005, the oleic acid present in the sebum is described as inducing the production of Malassezia, resulting in the formation of dandruff.

The need thus remains to find novel antidandruff agents which are effective without the disadvantages mentioned above, in particular not having an antifungal activity, and which can neutralize the action of oleic acid on the scalp and thus prevent the excessive colonization of the scalp by Malassezia sp.

The applicant company has now found, surprisingly, that the use of certain imprinted polymers of sol-gel type as defined below makes it possible to specifically trap the C₁₄-C₂₀ fatty acids which are the cause of the formation of dandruff, in particular oleic acid.

Thus, these specific molecularly imprinted polymers make it possible to trap the oleic acid present on the scalp and thus to prevent the colonization of the scalp by Malassezia microorganisms. They thus make it possible to reduce or prevent the appearance of dandruff.

A subject matter of the present invention is thus a molecularly imprinted polymer capable of being obtained according to a process comprising a first stage of polymerization of a mixture comprising:
i) one or more silane(s) of formula (I) defined below;
ii) one or more crosslinking agent(s) chosen from tetra(C₁-C₄)alkyl orthosilicates; and
iii) water;
iv) one or more porogenic solvent(s);
v) one or more C₁₄-C₂₀ fatty acid(s);

followed by a second stage of withdrawal of the C₁₄-C₂₀ fatty acid present in the polymer obtained on conclusion of the first stage.

Another subject matter of the invention is a process for the preparation of molecularly imprinted polymer as defined above.

Another subject matter of the invention is a cosmetic composition comprising, in a physiologically acceptable medium, a molecularly imprinted polymer as defined above.

Another subject matter of the invention is a cosmetic method for preventing and/or treating dandruff of the scalp, in particular that caused by yeasts of the Malassezia genus, characterized in that it comprises the application, to the scalp, of an imprinted polymer as defined above or of a cosmetic composition comprising it.

Another subject matter of the invention is the cosmetic use of imprinted polymer as defined above as active agent for preventing and/or treating dandruff of the scalp.

Molecularly imprinted polymers or MIPs are materials which are widely used for their applications in the fields of biotechnology, chemistry, chromatography, analytical chemistry and biology (J. Mol. Recognit., 19, 106-180 (2006); Molecularly Imprinted Materials: Science and Technology, Marcel Dekker, NY, M. Yan and O. Ramstrom (2005)). The concept of molecular imprinting relates to Emil Fisher's famous *"lock and key fit"* principle known since 1894 for enzymes with their ligand *(*Advances in Carbohydrate Chemistry and Biochemistry, 1-20 (1994)). Molecular imprinting consists more specifically in producing a polymer which comprises specific cavities in the shape and size of a target molecule or *"imprint",* also known as *template,* which serves as gage for the formation of recognition sites exhibiting a complementarity in shape with the imprinted molecule. Molecular imprints are polymers prepared from functional monomers polymerized around a molecule, also known as *"template".* The monomer is thus chosen so as to develop noncovalent interactions (hydrogen bond, electrostatic, ionic interactions, and nonionic, indeed even of low energy, such as Van der Waals bonds, or π-π stacking) with the template. The polymerization will subsequently be carried out in a *"porogenic"* solvent between the monomers complexed with the *template* and a crosslinking agent, so as to form specific cavities. The bonds between the *template* and the polymerized monomers (subsequent to hydrolysis, condensation) are subsequently broken by means of suitable solvents to extract the *template* from the polymer support. The extraction of the *template* molecule then leaves vacant recognition sites with a high affinity for the target molecule. The shape and the size of the imprint and also the spatial arrangement of the functional groups inside the recognition cavity are complementary to the template molecule and include sites of specific interactions with this same molecule.

This type of selective trapping is described in several scientific papers (see, for example, Analytical Chemistry, "Molecularly imprinted polymers: the next generation", 75(17), 376-383, (2003); Chemical Engineering Journal, "Selective separation of basic and reactive dyes by molecularly imprinted polymers (MIPs)", 149(1-3), 263-272, (2009), Kirk-Othmer Encyclopedia of Chemical Technology, "Molecular Imprinting", D. Spivak; accessible online since 06/25/2010, DOI: 10.1002/0471238961.molespiv.a01; *Molecularly Imprinted Polymers;* B.R. Hart and K.J. Shea, http://onlinelibrary.wiley.com/doi/10.1002/0471216275.esm054/full, Encyclopedia of Polymer, Science and Technology, accessible online since 07/15/2002; DOI: 10.1002/0471216275.esm054; J. Sep. Sci., M. Lasàkovà and P. Jandera, 32, 799-812).

The polymerization method used to manufacture the molecularly imprinted polymers according to the invention is the sol-gel polymerization process. The sol-gel process makes it possible to manufacture an inorganic polymer by simple chemical reactions known to a person skilled in the art (see, for example, Kirk-Othmer Encyclopedia of Chemical Technology, " Sol-Gel Technology", A. C. Pierre, placed online on 07/13/2007, DOI: 10.1002/0471238961.19151208051403.a01.pub2; http://onlinelibrary.wiley.com/doi/10.1002/0471238961.19151208051403.a01.pub2/pdf, and Ullmann's Encyclopedia of Industrial Chemistry, " Aerogels", N. Hüsing and U. Schubert, placed online on 12/15/2006, DOI: 10.1002/14356007.c01_c01.pub2: http://onlinelibrary.wiley.com/doi/10.1002/14356007.c01 c01.pub2/pdf). During the transformation of the reaction medium, the viscosity increases, changing from the "sol", which is defined as the colloidal suspension of very small particles, to a rigid and porous network, known as "gel".

The molecularly imprinted polymer is prepared from the silane of following formula (I):

R₁Si(OR₂)_{z}(R₃)ₓ (I)

in which:
- R₁ is a saturated or unsaturated, linear or branched and cyclic or acyclic C₁-C₆ hydrocarbon chain substituted by a group chosen from:
   - an amine NH₂ or NHR group, with R = C₁-C₄ alkyl,
   - an aryl or aryloxy group substituted by an amino group or by a C₁-C₄ aminoalkyl group,
      it being possible for R₁ to be interrupted in its chain by a heteroatom (O, S, NH) or a carbonyl (CO) group, R₁ being bonded to the silicon atom directly via a carbon atom,
- R₂ and R₃, which are identical or different, represent a linear or branched alkyl group comprising from 1 to 6 carbon atoms,
- z denotes an integer ranging from 1 to 3, and
- x denotes an integer ranging from 0 to 2,
with z+x = 3.

Preferably, R₂ represents an alkyl group comprising from 1 to 4 carbon atoms.
Preferably, R₂ represents a linear alkyl group comprising from 1 to 4 carbon atoms.
Preferably, R₂ represents the ethyl group.

Preferably, R₃ represents an alkyl group comprising from 1 to 4 carbon atoms.
Preferably, R₃ represents a linear alkyl group comprising from 1 to 4 carbon atoms.
Preferably, R₃ represents the methyl or ethyl group.

Preferably, R₁ is an acyclic chain.

Preferably, R₁ is a saturated or unsaturated and linear or branched C₁-C₆ hydrocarbon chain substituted by an amine NH₂ or NHR (R = C₁-C₆ alkyl, C₃-C₆ cycloalkyl or C₆ aromatic) group. Preferably, R₁ is a saturated linear C₁-C₆ hydrocarbon chain substituted by an amine NH₂ group. More preferably, R₁ is a saturated linear C₂-C₄ hydrocarbon chain substituted by an amine NH₂ group.

Preferably, R₁ is a saturated linear C₁-C₆ hydrocarbon chain substituted by an amine NH₂ group,
R₂ represents an alkyl group comprising from 1 to 4 carbon atoms,
R₃ represents an alkyl group comprising from 1 to 4 carbon atoms.

Preferably, z is equal to 3.

Preferably, the silane of formula (I) is chosen from 3-aminopropyltriethoxysilane (APTES), 2-aminoethyltriethoxysilane (AETES), 3-aminopropylmethyldiethoxysilane, N-(2-aminoethyl)-3-aminopropyltriethoxysilane, 3-(m-aminophenoxy)propyltrimethoxysilane, p-aminophenyltrimethoxysilane or N-(2-aminoethylaminomethyl)phenethyltrimethoxysilane. Preferably, the silane (I) is chosen from 3-aminopropyltriethoxysilane (APTES), 2-aminoethyltriethoxysilane (AETES), 3-aminopropylmethyldiethoxysilane or N-(2-aminoethyl)-3-aminopropyltriethoxysilane.
Preferably, the silane (I) is 3-aminopropyltriethoxysilane (APTES).

The sol-gel polymerization is carried out in the presence of a crosslinking agent chosen from tetra(C₁-C₄)alkyl orthosilicates. In particular, the crosslinking agent can be chosen from tetraethoxysilane (TEOS) or tetramethoxysilane (TMOS). Preferably, the crosslinking agent is tetraethoxysilane (TEOS).

The sol-gel polymerization is carried out in the presence of water in order to bring about the hydrolysis of the silane (I) and then its condensation.
The polymerization can be carried out in the presence of an acid catalyst, in particular in order to accelerate the condensation reaction, such as, for example, inorganic acids, such as hydrochloric acid, or organic acids, such as acetic acid.

The polymerization can be carried out in the presence of a basic catalyst, such as, for example, aqueous ammonia.

A list of the catalysts used to catalyze the sol-gel reaction is described in particular in the papers "Catalysts and the structure of SiO2 sol-gel films", Journal Of Materials Science, 35 (2000), 1835-184, and "Sol-gel processing of silica: II. The role of the catalyst", Journal of Non-Crystalline Solids, Volume 87, Issues 1-2, 2 October 1986, Pages 185-198.

The synthesis of this polymer is carried out according to chemical reactions known to a person skilled in the art which are triggered when the reactants are brought into contact with water and optionally with a catalyst which has the effect 1) of hydrolyzing the alkoxy (OR₂) groups of the silanes to give hydroxyl groups and then 2) of condensing the hydrolyzed products to result 3) in the polymerization of the system.

The process for the preparation of the molecularly imprinted polymers is advantageously carried out at a temperature of between 20 and 150°C inclusively.

Preferably, in the preparation process according to the invention, the C₁₄-C₂₀ fatty acid, the silane (I) and the crosslinking agent tetra(C₁-C₄)alkyl orthosilicate are employed according to a C₁₄-C₂₀ fatty acid/silane (I)/tetra(C₁-C₄)alkyl orthosilicate molar ratio ranging from 1/[1 to 20]/[1 to 40], preferably ranging from 1/[1 to 10]/[1 to 30] and preferentially ranging from 1/[1 to 5]/[1 to 5].

The molecularly imprinted sol-gel polymers are prepared from a porogenic solvent which preferably has a polarity which makes it possible i) to dissolve the C₁₄-C₂₀ fatty acid imprint molecule and/or ii) which is suitable for the interaction of said C₁₄-C₂₀ fatty acid imprint molecule with the molecularly imprinted polymer.
*"Porogenic"* solvent is understood to mean a solvent capable of creating a porous network able to convey the C₁₄-C₂₀ fatty acid molecules as far as the imprints of the polymer.
The porogenic solvent should also promote the C₁₄-C₂₀ fatty acid imprint molecule/monomer interactions and the stability of the complex formed.
According to a preferred form, when the dissolution of the imprint molecule in the prepolymerization mixture demands it, the porogenic solvent is chosen from polar protic organic solvents, such as water or C₁-C₈ alcohols, such as ethanol.
According to another preferred embodiment, the porogenic solvent is a polar aprotic solvent, such as acetonitrile, tetrahydrofuran (THF), dialkylformamides (dimethylformamide, diethylformamide), N-methyl-2-pyrrolidinone (NMP), N-ethyl-2-pyrrolidinone (NEP), N,N'-dimethylpropyleneurea (DMPU) and dimethyl sulfoxide (DMSO).
Use may also be made of a mixture of porogenic solvents.

Preferably, the porogenic solvent used according to the invention is a solvent chosen from polar (a)protic solvents, such as water, C₁-C₈ alcohols, such as ethanol, and acetonitrile, and their mixtures.

### The imprint molecules or Template:

The aim of the invention is to make available a molecularly imprinted polymer which captures saturated or unsaturated fatty C₁₄-C₂₀ carboxylic acids, in particular oleic acid, at the surface of the scalp.

As seen above, the fatty C₁₄-C₂₀ carboxylic acids *"template"* is a compound which mimics oleic acid, which causes dandruff, within the molecularly imprinted polymer in order for the molecularly imprinted polymer subsequently to be able to capture oleic acid when it is applied to the scalp.

Mention may be made, as saturated or unsaturated fatty C₁₄-C₂₀ carboxylic acid, of myristic acid (C14:0), myristoleic acid (C14:1), pentadecanoic acid (C15:0), palmitic acid (C16:0), palmitoleic acid (C16:1), sapienic acid (C16:1), heptadecanoic acid (or margaric acid) (C17:0), stearic acid (C18:0), oleic acid (C18:1), arachidic acid (C20:0) or eicosenoic acid (C20:1). Preferably, the fatty acid is oleic acid.

The process for the preparation of the imprinted polymer according to the invention comprises:
a first stage of polymerization of a mixture comprising:
   i) one or more silane(s) of formula (I) defined below;
   ii) one or more crosslinking agent(s) chosen from tetra(C₁-C₄)alkyl orthosilicates; and
   iii) water;
   iv) one or more porogenic solvent(s);
   v) one or more C₁₄-C₂₀ fatty acid(s);
followed by a second stage of withdrawal of the C₁₄-C₂₀ fatty acid present in the polymer obtained on conclusion of the first stage.

The withdrawal stage is carried out by washing the polymer obtained in the first stage with a washing solvent.
The washing solvent can be chosen from C₁-C₄ alcohols, water, acetonitrile, tetrahydrofuran (THF), dialkylformamides (dimethylformamide, diethylformamide), N-methyl-2-pyrrolidinone (NMP), N-ethyl-2-pyrrolidinone (NEP), N,N'-dimethylpropyleneurea (DMPU), dimethyl sulfoxide (DMSO), chloroform, acetic acid, aqueous ammonia, diethylamine, and their mixtures.

After the washing, the imprinted polymer no longer comprises the C₁₄-C₂₀ fatty acid.

The empty imprints thus make it possible for the polymer to be able to capture oleic acid when it is applied to the scalp.

### Characterization of the MIP

The characterization of the MIP consists in demonstrating the formation of the imprints and in evaluating their number and their affinity for the targeted molecule. These results can be complemented by a study of the morphology of the material (size and shape of the particles, porosity and specific surface). These methods are known to a person skilled in the art (see, for example, point 1.7, p. 49, of the June 2010 doctoral thesis of R. Walsh, *Development and Characterization of MIP* http://repository.wit.ie/1619/1/Development and characterisation of molecularly imprinted suspension polymers.pdf)

The cosmetic composition according to the invention comprises the molecularly imprinted polymer as described above and a physiologically acceptable medium.
The term "physiologically acceptable medium" is understood to mean a medium compatible with cutaneous tissues, such as the skin and the scalp.

The molecularly imprinted polymer according to the invention can be present in the cosmetic composition in a content ranging from 0.1% to 20% by weight, preferably ranging from 0.1% to 10% by weight and preferentially ranging from 0.1% to 5% by weight, with respect to the total weight of the composition.

The physiologically acceptable medium of the composition can be more particularly composed of water and optionally of a physiologically acceptable organic solvent chosen, for example, from lower alcohols comprising from 2 to 8 carbon atoms and in particular from 2 to 6 carbon atoms, such as ethanol, isopropanol, propanol or butanol, polyethylene glycols having from 6 to 80 ethylene oxide units, and polyols, such as propylene glycol, isoprene glycol, butylene glycol, glycerol and sorbitol.

The compositions according to the invention can be provided in all the formulation forms conventionally used for a topical application and in particular in the form of aqueous or aqueous/alcoholic solutions, of oil-in-water (O/W), water-in-oil (W/O) or multiple (triple: W/O/W or O/W/O) emulsions, of aqueous gels or of dispersions of a fatty phase in an aqueous phase using spherules, it being possible for these spherules to be polymeric nanoparticles, such as nanospheres and nanocapsules, or lipid vesicles of ionic and/or nonionic type (liposomes, niosomes or oleosomes). These compositions are prepared according to the usual methods.

In addition, the compositions used according to the invention can be more or less fluid and can have the appearance of a white or colored cream, an ointment, a milk, a lotion, a serum, a paste, a mousse or a shampoo.

The composition used according to the invention comprise adjuvants commonly employed in the cosmetics field and chosen in particular from water, oils, waxes, pigments, fillers, dyes, surfactants, emulsifiers, cosmetic active agents, UV-screening agents, polymers, thickeners, film-forming polymers, preservatives, fragrances, bactericides, odor absorbers or antioxidants.
The amounts of these various adjuvants are those conventionally used in the field under consideration, for example from 0.01 % to 20% of the total weight of the composition.

### Additional antidandruff active agents

The composition according to the invention can comprise an additional antidandruff active agent chosen in particular from ellagic acid and its ethers, salts of ellagic acid and its ethers, pyrithione salts, 1-hydroxy-2-pyridone derivatives and selenium (poly)sulfides, and also their mixtures.

Ellagic acid, or 2,3,7,8-tetrahydroxy[1]benzopyrano[5,4,3-cde][1]benzopyran-5,10-dione, is a well-known molecule which is present in the plant kingdom. Reference may be made to the publication of the Merck Index, 20th edition (1996), No. 3588.
Ellagic acid exhibits the following chemical formula: 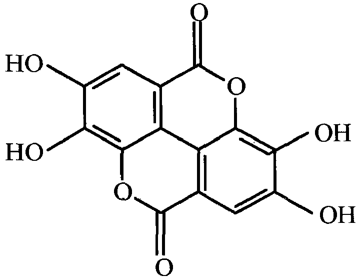 which comprises four fused rings.

The ellagic acid ether(s) which can be used according to the invention are preferably chosen from the mono-, di-, tri- or polyethers obtained by etherification of one or more hydroxyl groups (one of the four OH groups of ellagic acid) of ellagic acid to give one or more OR groups, R being chosen from C₂-C₂₀ alkyl groups, polyoxyalkylene groups and in particular polyoxyethylene and/or polyoxypropylene groups, and groups derived from one or more mono- or polysaccharides, such as, for example, the group of following formula:

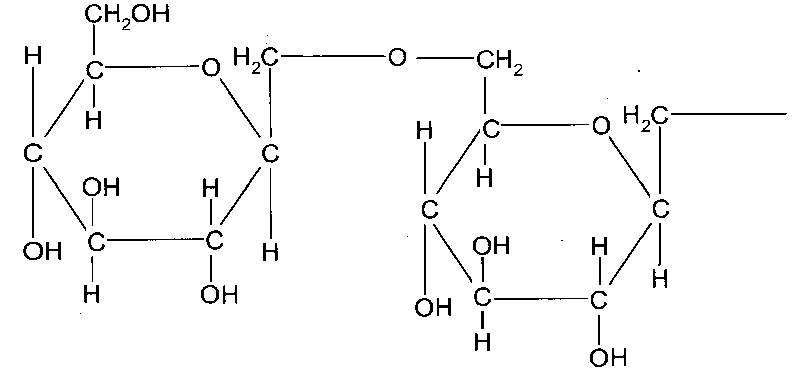

In the case of the di-, tri- or polyethers of ellagic acid, the R groups as defined above can be identical or different.
Preferably, these ethers of ellagic acid are chosen from 3,4-di-O-methyl ellagic acid, 3,3',4-tri-O-methyl ellagic acid and 3,3'-di-O-methyl ellagic acid.
The salt(s) of ellagic acid and/or of its ethers which can be used according to the invention are preferably chosen from alkali metal or alkaline earth metal salts, such as the sodium, potassium, calcium and magnesium salt, the ammonium salt and the salts of amines, such as triethanolamine, monoethanolamine, arginine and lysine salts. Preferably, the salt(s) of ellagic acid and/or of its ethers which can be used according to the invention are chosen from alkali metal or alkaline earth metal salts, in particular the sodium, potassium, calcium or magnesium salts.

Pyrithione is the compound 1-hydroxy-2(1H)-pyridinethione or 2-pyridinethiol 1-oxide.
The pyrithione salts capable of being used in the context of the invention are in particular the monovalent metal salts and the divalent metal salts, such as the sodium, calcium, magnesium, barium, strontium, zinc, cadmium, tin and zirconium salts. The divalent metal salts and in particular the zinc salt (zinc pyrithione) are particularly preferred.

The 1-hydroxy-2-pyridone derivatives are preferably chosen from the compounds of formula (A1) or their salts: 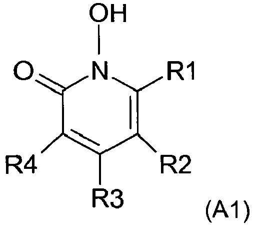 in which:
- R1 denotes a hydrogen atom; a linear or branched alkyl group having from 1 to 17 carbon atoms; a cycloalkyl group having from 5 to 8 carbon atoms; a cycloalkyl-alkyl group, the cycloalkyl group having from 5 to 8 carbon atoms and the alkyl group having from 1 to 4 carbon atoms; an aryl or aralkyl group, the aryl group having from 6 to 30 carbon atoms and the alkyl group having from 1 to 4 carbon atoms; an aryl-alkenyl group, the aryl group having from 6 to 30 carbon atoms and the alkenyl group having from 2 to 4 carbon atoms; it being possible for the cycloalkyl and aryl groups as defined above to be substituted by one or more alkyl groups having from 1 to 4 carbon atoms or else one or more alkoxy groups having from 1 to 4 carbon atoms;
- R2 denotes a hydrogen atom; an alkyl group having from 1 to 4 carbon atoms; an alkenyl group having from 2 to 4 carbon atoms; a halogen atom or a benzyl group;
- R3 denotes a hydrogen atom; an alkyl group having from 1 to 4 carbon atoms or a phenyl group; and
- R4 denotes a hydrogen atom; an alkyl group having from 1 to 4 carbon atoms; an alkenyl group having from 2 to 4 carbon atoms; a methoxymethyl group; a halogen atom or a benzyl group.

Among these compounds, those which are particularly preferred consist of 1-hydroxy-4-methyl-6-(2,4,4-trimethylpentyl)-2(1H)-pyridone and 6-cyclohexyl-1-hydroxy-4-methyl-2(1 H)-pyridone.
Mention may be made, among the salts which can be used, of the salts of lower (C₁-C₄) alkanolamines, such as ethanolamine and diethanolamine, amine or alkylamine salts, and also the salts with inorganic cations, such as ammonium salts and the salts of alkali metals or alkaline earth metals.
Preference will very particularly be given to the monoethanolamine salt of 1-hydroxy-4-methyl-6-(2,4,4-trimethylpentyl)-2(1H)-pyridinone (or piroctone), more commonly referred to as piroctone olamine or octopirox.

Mention may be made, among the selenium (poly)sulfides, of selenium disulfide and the selenium polysulfides of formula SeₓS_{y} in which x and y are numbers such that x + y = 8. Selenium disulfide is provided in the form of a powder, the particles of which generally have a particle size of less than 200 µm and preferably of less than 25 µm.

Preferably, the antidandruff agent is chosen from ellagic acid, zinc pyrithione, piroctone olamine and selenium disulfide, and also their mixture.

The additional antidandruff active agents can be present in the composition according to the invention in a proportion of from 0.001% to 30% by weight and preferably in a proportion of from 0.5% to 25% by weight, with respect to the total weight of the composition.

The examples below illustrate the invention without, however, limiting the scope thereof.

### EXAMPLES

### Syntheses of the molecularly imprinted polymers (or MIPs)

### Example 1 (invention) and Example 2 (outside the invention):

**Reactants and solvents used:**

| | Template (Oleic acid) | APTES | TEOS | H₂O | Ethanol | HCl (at 35% by weight in water) |
|---|---|---|---|---|---|---|
| Example 1 (invention) | 282.46 mg | 1.87 ml | 6.69 ml | 1.04 ml | 4.44 ml | 1.48 ml |
| Example 2 (outside the invention) | 0 | 1.87 ml | 6.69 ml | 1.04 mL | 4.44 mL | 1.48 ml |

| | | | | | | |
|---|---|---|---|---|---|---|
| APTES: (3-aminopropyl)triethoxysilane; TEOS: tetraethyl orthosilicate | | | | | | |

Example 1: The reactants and solvents were mixed in a beaker and then stirred at 60°C overnight. The reaction mixture was filtered and the precipitate was dried in an oven at 100°C overnight. After polymerization and attainment of the polymer impregnated with oleic acid, the impregnated polymer was washed 3 times with a 0.1 M ammonium hydroxide solution at 60°C and twice with methanol. Subsequently, the imprinted polymer, thus emptied of oleic acid, was dried under vacuum overnight. An oleic acid-imprinted polymer (MIP ex. 1) was obtained in the form of opaque white spherical particles.
The mean diameter of the particles obtained is 1081 nm (determined by diffraction light scattering (DLS)).

Example 2: The same synthesis was carried out in the absence of the template (oleic acid) in order to prepare a non-imprinted polymer (NIP). This acts as reference (non-selective polymer). An opaque white polymer was obtained in the form of spherical particles. The mean diameter of the particles obtained is 1261 nm.

### Examples 3 (invention) and 4 (outside the invention):

**Reactants and solvents used:**

| | Template (Oleic acid) | APTES | TEOS | H₂O | Ethanol |
|---|---|---|---|---|---|
| Example 3 (invention) | 400 µl | 0.5 ml | 0.5 ml | 0.5 ml | 10 ml |
| Example 4 (outside the invention) | 0 | 0.5 ml | 0.5 ml | 0.5 ml | 10 ml |

| | | | | | |
|---|---|---|---|---|---|
| The same synthesis was carried out as for examples 1 and 2, except that the polymerization is carried out at ambient temperature for 6 days. For example 4, 90 µl of acetic acid were added to bring about the reaction. | | | | | |

An oleic acid-imprinted polymer (MIP ex. 3) and an imprint-free polymer (NIP ex. 4) were obtained in the form of opaque white spherical particles.

The mean diameters of the particles are 332 nm (example 3) and 296 nm (example 4).

### Examples 5 (invention) and 6 (outside the invention):

**Reactants and solvents used:**

| | Template (Oleic acid) | APTES | TEOS | H₂O | Ethanol |
|---|---|---|---|---|---|
| Example 5 (invention) | 400 µl | 0.5 ml | 0.5 ml | 0.5 ml | 10 ml |
| Example 6 (outside the invention) | 0 | 0.5 ml | 0.5 ml | 0.5 ml | 10 ml |

| | | | | | |
|---|---|---|---|---|---|
| The same synthesis was carried out as for examples 1 and 2, except that the polymerization was carried out at 40°C overnight. For example 6, 90 µl of acetic acid were added to bring about the reaction. An oleic acid-imprinted polymer (MIP ex. 5) and an imprint-free polymer (NIP ex. 6) were obtained in the form of opaque white spherical particles. | | | | | |

### Example 7: Recognition test

The polymers obtained in the examples described above were suspended in a 5/55/40 (mixture by volume) propylene glycol/ethanol/water solution. Increasing concentrations of polymers were introduced into 2 ml polypropylene tubes, and [³H]-oleic acid (0.45 nM, 15 nanoCuries) was added. The final volume was adjusted to 1 ml. The tubes were incubated overnight at ambient temperature on a rotary shaker. They were subsequently centrifuged at 16 000 g for 15 min and a 500 µl aliquot of the supernatant was withdrawn and transferred into a scintillation vial containing 3 ml of liquid scintillant (reference 327123 from Fluka). The amount of free radioligand was assayed by a scintillation counter (Beckman LS-6000 IC). This amount was compared with that of the solution of the [³H]-oleic acid before it is brought into contact with the polymers. The difference makes it possible to evaluate the amount of [³H]-oleic acid adsorbed.

The following results were obtained:

### MIP of example 1 (invention) and NIP of example 2 (outside the invention)

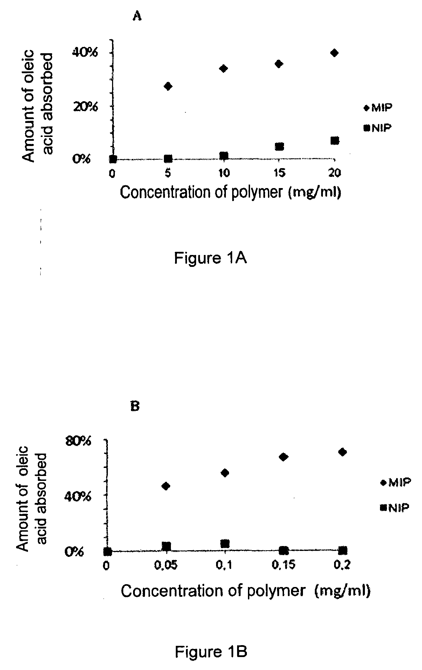

Figure 1A below shows the variation in the amount of oleic acid adsorbed as a function of the concentration of polymer (MIP of example 1; NIP of example 2).

The results obtained show that the MIP of example 1 (invention) is capable of better recognising the oleic acid (greater amount of oleic acid adsorbed) than the NIP of example 2 (outside the invention).

### MIP of example 3 (invention) and NIP of example 4 (outside the invention)

Figure 1 B below shows the change in the amount of oleic acid adsorbed as a function of the concentration of polymer (MIP of example 3; NIP of example 4).

The results obtained show that the MIP of example 3 (invention) is capable of better recognising the oleic acid (greater amount of oleic acid adsorbed) than the NIP of example 4 (outside the invention).

### Example 8: Recognition at the surface of the stratum corneum

The following solutions were prepared:
Solution A1: 10 mg/ml of the MIP of example 5 were suspended in a propylene glycol/ethanol/water solution: 5/55/40.
Solution A2: 10 mg/ml of the NIP of example 6 were suspended in a propylene glycol/ethanol/water solution: 5/55/40.
Solution B: A 14 mM solution of non-radiolabelled oleic acid in propylene glycol/ethanol/water: 5/55/40.
Solution C: A solution of [³H]-oleic acid (3 µl - Activity: 1 mCi/ml, specific activity: 73 Ci/mmol and purchased from Sigma-Aldrich) in ethanol (10 ml).
Solution D: A mixture of solution B (100 µl) and solution C (100 µl).

3 pieces of human stratum corneum (1 cm²) were placed on glass slides (1.4 cm²) with the hydrophobic face of the stratum corneum at the top (toward the surface). The following solutions were added to these strata cornea:
Stratum corneum No. 1: 2 µl of solution D (control)
Stratum corneum No. 2: 4 µl of solution A1 then 2 µl of solution D (invention)
Stratum corneum No. 3: 4 µl of solution A2 then 2 µl of solution D (outside the invention)

The treated samples of stratum corneum were left in a closed petri dish for 3 hours and then washed twice with a propylene glycol/ethanol/water 5/55/40 solution (two times 2 ml) and then twice with a 5% by weight aqueous sodium lauryl sulfate solution (two times 1.5 ml). Subsequently, the pieces of stratum corneum were completely digested by Soluene®-350 solutions (1 ml, purchased from Sigma-Aldrich). Digestion took place at 40°C for 1.5 hours. The solutions obtained were added to scintillation solutions (5 ml, reference 327123 from Sigma-Aldrich) and the radioactivity was measured in a scintillation counter. Digestion is necessary in order to prevent interactions between the stratum corneum and the radiolabelled oleic acid, which can reduce the radioactivity measured.

The following results were obtained:

**Table 1**

| | Sample No. 1 (control) | Sample No. 2 (invention) | Sample No. 3 (outside the invention) |
|---|---|---|---|
| Radioactivity measured in the stratum corneum | 363.3 | 87.0 | 339.4 |
| (in disintegrations per minute) | | | |

The results obtained are interpreted in the following way: The higher the number of disintegrations per minute detected by the scintillation counter, the greater the radioactivity of the solution. A measured number of disintegrations per minute close to that of the control is interpreted as corresponding to no inhibition of the diffusion of the oleic acid in the stratum corneum. In this case, the oleic acid is not trapped by the polymer.

A measured number of disintegrations per minute which is lower than that of the control is interpreted as a reduction in the diffusion of the oleic acid in the stratum corneum: it reflects the trapping of the oleic acid at the surface of the stratum corneum by the MIP.

In theory, sample No. 1 (the control) should have the highest radioactivity. The NIP (sample No. 3) should not trap/inhibit the diffusion of the oleic acid in the skin and should thus have a figure close to or identical to sample No. 1. The MIP (sample No. 2) should have the least radioactivity as it was designed to trap the oleic acid.

The results obtained which appear in table 1 show that there is a significant reduction in the radioactivity in the stratum corneum after a pretreatment with an MIP of the invention (example 5), which confirms that the oleic acid is trapped at the surface of the stratum corneum by the MIP tested.

### Example 9: Antidandruff shampoo

An antidandruff shampoo is prepared which comprises the following ingredients:

| | |
|---|---|
| Sodium lauryl ether sulfate (2.2 OE) as an aqueous solution | 17 g AM |
| (Texapon AOS 225 UP from Cognis) | |
| Coco-betaine as an aqueous solution (Dehyton AB 30 from Cognis) | 2.5 g AM |
| Coconut acid monoisopropanolamide (Rewomid V 3203 from Goldschmidt) | 2.0 g |
| | |
| Molecularly imprinted polymer of example 1 | 1 g AM |
| Preservatives | 1.1 g |
| Fragrance | 0.5 |
| Water | q.s. for 100 g |

The shampoo, applied to the hair and the scalp, makes it possible to alleviate the appearance of dandruff.

### Example 10: Antidandruff lotion

An antidandruff lotion is prepared which comprises the following ingredients:
- Molecularly imprinted polymer of example 3 0.3 g AM
- Preservatives q.s.
- Water q.s. for 100 g

The lotion, applied to the hair and the scalp, makes it possible to alleviate the appearance of dandruff.

A similar composition is prepared with the polymer of example 5.

## Claims

1. A process for the preparation of a molecularly imprinted polymer comprising a first stage of polymerization of a mixture comprising:
i) one or more silane(s);
ii) one or more crosslinking agent(s) chosen from tetra(C₁-C₄)alkyl orthosilicates; and
iii) water;
iv) one or more porogenic solvent(s);
v) one or more C₁₄-C₂₀ fatty acid(s);
followed by a second stage of withdrawal of the C₁₄-C₂₀ fatty acid present in the polymer obtained on conclusion of the first stage,
the silane corresponding to the following formula (I):
R₁Si(OR₂)_{z}(R₃)ₓ (I)
in which:
• R₁ is a saturated or unsaturated, linear or branched and cyclic or acyclic C₁-C₆ hydrocarbon chain substituted by a group chosen from:
- an amine NH₂ or NHR group, with R = C₁-C₄ alkyl,
- an aryl or aryloxy group substituted by an amino group or by a C₁-C₄ aminoalkyl group,
it being possible for R₁ to be interrupted in its chain by a heteroatom (O, S, NH) or a carbonyl (CO) group, R₁ being bonded to the silicon atom directly via a carbon atom,
• R₂ and R₃, which are identical or different, represent a linear or branched alkyl group comprising from 1 to 6 carbon atoms,
• z denotes an integer ranging from 1 to 3, and
• x denotes an integer ranging from 0 to 2,
with z+x = 3.

2. The process as claimed in the preceding claim, **characterized in that**, for the silane (I):
R₁ is a saturated linear C₁-C₆ hydrocarbon chain substituted by an amine NH₂ group,
R₂ represents an alkyl group comprising from 1 to 4 carbon atoms,
R₃ represents an alkyl group comprising from 1 to 4 carbon atoms.

3. The process as claimed in either of the preceding claims, **characterized in that**, for the silane (I), z is equal to 3.

4. The process as claimed in one of the preceding claims, **characterized in that** the silane of formula (I) is chosen from 3-aminopropyltriethoxysilane (APTES), 2-aminoethyltriethoxysilane (AETES), 3-aminopropylmethyldiethoxysilane, N-(2-aminoethyl)-3-aminopropyltriethoxysilane, 3-(m-aminophenoxy)propyltrimethoxysilane, p-aminophenyltrimethoxysilane or N-(2-aminoethylaminomethyl)phenethyltrimethoxysilane.

5. The process as claimed in one of the preceding claims, **characterized in that** the silane (I) is 3-aminopropyltriethoxysilane (APTES).

6. The process as claimed in one of the preceding claims, **characterized in that** the tetra(C₁-C₄)alkyl orthosilicate is tetraethoxysilane (TEOS).

7. The process as claimed in one of the preceding claims, **characterized in that** the C₁₄-C₂₀ fatty acid is oleic acid.

8. The process as claimed in one of the preceding claims, **characterized in that** be carried out in the presence of an acid catalyst or of a basic catalyst.

9. The process as claimed in one of the preceding claims, **characterized in that** the C₁₄-C₂₀ fatty acid, the silane (I) and tetra(C₁-C₄)alkyl orthosilicate are employed according to a C₁₄-C₂₀ fatty acid/silane (I)/tetra(C₁-C₄)alkyl orthosilicate molar ratio ranging from 1/[1 to 20]/[1 to 40], preferably ranging from 1/[1 to 10]/[1 to 30] and preferentially ranging from 1/[1 to 5]/[1 to 5].

10. The process as claimed in one of the preceding claims, **characterized in that** the stage of withdrawal of the fatty acid is carried out by washing the polymer obtained in the first stage with a washing solvent chosen from C₁-C₄ alcohols, water, acetonitrile, tetrahydrofuran (THF), dialkylformamides (dimethylformamide, diethylformamide), N-methyl-2-pyrrolidinone (NMP), N-ethyl-2-pyrrolidinone (NEP), N,N'-dimethylpropyleneurea (DMPU), dimethyl sulfoxide (DMSO), chloroform, acetic acid, aqueous ammonia, diethylamine, and their mixtures.

11. A molecularly imprinted polymer capable of being obtained according to the preparation process as claimed in any one of the preceding claims.

12. A cosmetic composition comprising, in a physiologically acceptable medium, a molecularly imprinted polymer as claimed in claim 11.

13. A nontherapeutic cosmetic method for preventing and/or treating dandruff of the scalp, in particular that caused by yeasts of the Malassezia genus, **characterized in that** it comprises the application, to the scalp, of an imprinted polymer as claimed in claim 11 or of a cosmetic composition comprising it.

14. The nontherapeutic cosmetic use of imprinted polymer as claimed in claim 11 as active agent for preventing and/or treating dandruff of the scalp.

## Patentansprüche

1. Verfahren zur Herstellung eines molekular geprägten Polymers, umfassend einen ersten Schritt des Polymerisierens einer Mischung, umfassend:
i) ein oder mehrere Silan(e);
ii) ein oder mehrere Vernetzungsmittel, ausgewählt aus Tetra(C₁-C₄)-Alkylorthosilikaten; und
iii) Wasser;
iv) ein oder mehrere porogene(s) Lösungsmittel;
v) eine oder mehrere C₁₄-C₂₀-Fettsäure(n);
gefolgt von einem zweiten Schritt des Entfernens der in dem zu Beendigung des ersten Schritts erhaltenen Polymer vorliegenden C₁₄-C₂₀-Fettsäure,
wobei das Silan der folgenden Formel (I) entspricht;
R₁Si(OR₂)₂(R₃)ₓ (I)
in der:
• R₁ eine gesättigte oder ungesättigte, geradkettige oder verzweigte und cyclische oder acyclische C₁-C₆-Kohlenwasserstoffkette ist, die durch eine Gruppe, ausgewählt aus den Folgenden, substituiert ist:
- einer Amin-NH₂- oder NHR-Gruppe, wobei R = C₁-C₄-Alkyl,
- einer Aryl- oder Aryloxygruppe, die durch eine Aminogruppe oder durch eine C₁-C₄-Aminoalkylgruppe substituiert ist,
wobei R₁ in seiner Kette durch ein Heteroatom (O, S, NH) oder eine Carbonyl(CO)-Gruppe unterbrochen sein kann, wobei R₁ direkt über ein Kohlenstoffatom an das Siliziumatom gebunden ist,
• R₂ und R₃, die gleich oder verschieden sind, eine geradkettige oder verzweigte Alkylgruppe mit 1 bis 6 Kohlenstoffatomen bedeuten,
• z eine ganze Zahl von 1 bis 3 bedeutet, und
• x eine ganze Zahl von 0 bis 2 bedeutet,
wobei z+x = 3.

2. Verfahren nach dem vorhergehenden Anspruch, **dadurch gekennzeichnet, dass** für das Silan (I):
R₁ eine gesättigte geradkettige C₁-C₆-Kohlenwasserstoffkette, die durch eine Amin-NH₂-Gruppe substituiert ist, bedeutet,
R₂ eine Alkylgruppe mit 1 bis 4 Kohlenstoffatomen bedeutet,
R₃ eine Alkylgruppe mit 1 bis 4 Kohlenstoffatomen bedeutet.

3. Verfahren nach einem der vorhergehenden Ansprüche, **dadurch gekennzeichnet, dass** für das Silan (I) z gleich 3 ist.

4. Verfahren nach einem der vorhergehenden Ansprüche, **dadurch gekennzeichnet, dass** das Silan der Formel (I) aus 3-Aminopropyltriethoxysilan (APTES), 2-Aminoethyltriethoxysilan (AETES), 3-Aminopropylmethyldiethoxysilan, N-(2-Aminoethyl)-3-aminopropyltriethoxy-silan, 3-(m-Aminophenoxy)propyltrimethoxysilan, p-Aminophenyltrimethoxysilan oder N-(2-Aminoethylaminomethyl)phenethyltrimethoxysilan ausgewählt ist.

5. Verfahren nach einem der vorhergehenden Ansprüche, **dadurch gekennzeichnet, dass** das Silan (I) 3-Aminopropyltriethoxysilan (APTES) ist.

6. Verfahren nach einem der vorhergehenden Ansprüche **dadurch gekennzeichnet, dass** das Tetra(C₁-C₄)-alkylorthosilikat Tetraethoxysilan (TEOS) ist.

7. Verfahren nach einem der vorhergehenden Ansprüche, **dadurch gekennzeichnet, dass** die C₁₄-C₂₀-Fettsäure Ölsäure ist.

8. Verfahren nach einem der vorhergehenden Ansprüche, **dadurch gekennzeichnet, dass** es in Gegenwart eines sauren Katalysators oder eines basischen Katalysators durchgeführt werden kann.

9. Verfahren nach einem der vorhergehenden Ansprüche, **dadurch gekennzeichnet, dass** die C₁₄-C₂₀-Fettsäure, das Silan (I) und das Tetra(C₁-C₄)-alkylorthosilikat in einem molaren Verhältnis von C₁₄-C₂₀-Fettsäure/Silan(I)/Tetra(C₁-C₄)-alkylorthosilikat im Bereich von 1/[1 bis 20]/[1 bis 40], vorzugsweise im Bereich von 1/[1 bis 10]/[1 bis 30] und bevorzugt im Bereich von 1/[1 bis 5]/[1 bis 5] eingesetzt werden.

10. Verfahren nach einem der vorhergehenden Ansprüche, **dadurch gekennzeichnet, dass** der Schritt des Entfernens der Fettsäure durch Waschen des im ersten Schritt erhaltenen Polymers mit einem Waschlösungsmittel, ausgewählt aus C₁-C₄-Alkoholen, Wasser, Acetonitril, Tetrahydrofuran (THF), Dialkylformamiden (Dimethylformamid, Diethylformamid), N-Methyl-2-pyrrolidinon (NMP), N-Ethyl-2-pyrrolidinon (NEP), N,N'-Dimethylpropylenharnstoff (DMPU), Dimethylsulfoxid (DMSO), Chloroform, Essigsäure, Ammoniak, Diethylamin und Mischungen davon, erfolgt.

11. Molekular geprägtes Polymer, das nach dem Herstellungsverfahren nach einem der vorhergehenden Ansprüche erhältlich ist.

12. Kosmetikzusammensetzung, die ein molekular geprägtes Polymer nach Anspruch 11 in einem physiologisch unbedenklichen Medium umfasst.

13. Kosmetisches nichttherapeutisches Verfahren zum Vorbeugen und/oder Behandeln von Kopfhautschuppen, insbesondere solchen, die von Hefen der Gattung Malassezia verursacht werden, **dadurch gekennzeichnet, dass** es die Aufbringung eines geprägten Polymers nach Anspruch 11 oder einer Kosmetikzusammensetzung, die es umfasst, auf die Kopfhaut umfasst.

14. Kosmetische nichttherapeutische Verwendung eines geprägten Polymers nach Anspruch 11 als Wirkstoff zum Vorbeugen und/oder Behandeln von Kopfhautschuppen.

## Revendications

1. Procédé de préparation d'un polymère à empreinte moléculaire comprenant une première étape de polymérisation d'un mélange comprenant :
i) un ou plusieurs silane(s) ;
ii) un ou plusieurs agent(s) de réticulation choisi(s) parmi les tétraalkyl(C₁-C₄)orthosilicates ; et
iii) de l'eau ;
iv) un ou plusieurs solvant(s) porogène(s) ;
v) un ou plusieurs acide(s) gras en C₁₄-C₂₀ ;
suivie d'une deuxième étape de retrait de l'acide gras en C₁₄-C₂₀ présent dans le polymère obtenu à l'issue de la première étape,
le silane répondant à la formule (I) suivante :
R₁Si(OR₂)_{z}(R₃)ₓ (I)
dans laquelle :
• R₁ est une chaîne hydrocarbonée en C₁-C₆, linéaire ou ramifiée, saturée ou insaturée, cyclique ou acyclique substituée par un groupement choisi parmi les groupements :
- amine NH₂ ou NHR avec R= alkyle en C₁-C₄,
- un groupement aryle ou aryloxy substitué par un groupement amino ou par un groupement aminoalkyl en C₁-C₄ ;
R₁ pouvant être interrompu dans sa chaîne par un hétéroatome (O, S, NH) ou un groupement carbonyle (CO), R₁ étant lié à l'atome de silicium directement via un atome de carbone,
• R₂ et R₃ identiques ou différents, représentent un groupe alkyle linéaire ou ramifié, comprenant de 1 à 6 atomes de carbone,
• z désigne un nombre entier allant de 1 à 3, et
• x désigne un nombre entier allant de 0 à 2,
avec z+x =3.

2. Procédé selon la revendication précédente, **caractérisé en ce que** pour le silane (I) :
R₁ est une chaîne hydrocarbonée en C₁-C₆, linéaire saturée substituée par un groupement amine NH₂,
R₂ représente un groupe alkyle comprenant de 1 à 4 atomes de carbone,
R₃ représente un groupe alkyle comprenant de 1 à 4 atomes de carbone.

3. Procédé selon l'une des revendications précédentes, **caractérisé en ce que** pour le silane (I) z est égal à 3.

4. Procédé selon l'une des revendications précédentes, **caractérisé en ce que** le silane de formule (I) est choisi parmi le 3-aminopropyltriéthoxysilane (APTES), le 2-aminoéthyltriéthoxysilane (AETES), le 3-aminopropylméthyldiéthoxysilane, le N-(2-aminoéthyl)-3-aminopropyltriéthoxysilane, le 3-(m-aminophénoxy)propyltriméthoxysilane, le p-aminophényltriméthoxysilane, le N-(2-aminoéthylaminométhyl)phénéthyltriméthoxysilane.

5. Procédé selon l'une des revendications précédentes, **caractérisé en ce que** le silane (I) est le 3-aminopropyltriéthoxysilane (APTES).

6. Procédé selon l'une des revendications précédentes, **caractérisé en ce que** le tétraalkyl(C₁-C₄)orthosilicate est le tétraéthoxysilane (TEOS).

7. Procédé selon l'une des revendications précédentes, **caractérisé en ce que** l'acide gras en C₁₄-C₂₀ est l'acide oléique.

8. Procédé selon l'une des revendications précédentes, **caractérisé en ce qu'**il est effectué en présence d'un catalyseur acide ou d'un catalyseur basique.

9. Procédé selon l'une des revendications précédentes, **caractérisé en ce que** l'acide gras C₁₄-C₂₀, le silane (I) et tétraalkyl(C₁-C₄)orthosilicate sont mis en oeuvre selon un rapport molaire acide gras C₁₄-C₂₀ / silane (I) / tétraalkyl(C₁-C₄)orthosilicate allant de 1/ [1 à 20] / [1 à 40], de préférence allant de 1/ [1 à 10] / [1 à 30], préférentiellement allant de 1 / [1 à 5] / [1 à 5].

10. Procédé selon l'une des revendications précédentes, **caractérisé en ce que** l'étape de retrait de l'acide gras est effectuée par lavage du polymère obtenu à la première étape avec un solvant de lavage choisi parmi les alcools en C₁-C₄, l'eau, l'acétonitrile, le tétrahydrofurane (THF), les dialkylformamide (diméthylformamide, diéthylformamide), le N-méthyl-2-pyrrolidinone (NMP), le N-éthyl-2-pyrrolidinone (NEP), le N,N'-diméthylpropylène-urée (DMPU), le diméthylsulfoxyde (DMSO), le chloroforme, l'acide acétique, l'ammoniaque, la diéthylamine, et leurs mélanges.

11. Polymère à empreinte moléculaire susceptible d'être obtenu selon le procédé de préparation selon l'une quelconque des revendications précédentes.

12. Composition cosmétique comprenant, dans un milieu physiologiquement acceptable, un polymère à empreinte moléculaire selon la revendication 11.

13. Procédé cosmétique non thérapeutique pour prévenir et/ou traiter les pellicules du cuir chevelu, notamment celles provoquées par les levures du genre Malassezia, **caractérisé en ce qu'**il comprend l'application, sur le cuir chevelu, d'un polymère à empreinte selon la revendication 11 ou d'une composition cosmétique le comprenant.

14. Utilisation cosmétique non thérapeutique de polymère à empreinte selon la revendication 11 comme ingrédient actif pour prévenir et/ou traiter les pellicules du cuir chevelu.
